# FASCICULE DE BREVET EUROPEEN

(11) **EP 1 137 376 B1**
(45) Date de publication et mention de la délivrance du brevet: **06.09.2006**
(21) Numéro de dépôt: 99958286.9
(22) Date de dépôt: 09.12.1999
(51) Int. Cl.: A61F 2/44

(54) **PROTHESE DE DISQUE INTERVERTEBRAL A ENCEINTE DE LIQUIDE**
BANDSCHEIBENPROTHESE MIT FLÜSSIGKEITSGEFÜLLTER KAMMER
INTERVERTEBRAL DISC PROSTHESIS WITH LIQUID CHAMBER

(30) Priorité: 11.12.1998 FR 9815673
(43) Date de publication de la demande: 04.10.2001
(73) Titulaire: Stryker Spine, 33610 Cestas (FR)
(72) Inventeur: GAUCHET, Fabien, 60800 Duvy (FR)
(74) Mandataire: Le Forestier, Eric
(86) Numéro de dépôt international: PCT/FR1999/003074
(87) Numéro de publication internationale: WO 2000/035386

(56) Documents cités:
- EP-A- 0 277 282
- WO-A-96/01598
- DE-U- 9 000 094
- US-A- 4 863 477
- US-A- 5 534 028

## Description

L'invention concerne les prothèses de disque intervertébral.

On connaît, par exemple du document EP-0 277 282, une prothèse de disque intervertébral destinée à remplacer un disque intervertébral. Elle comporte deux plateaux aptes à venir en appui contre les plateaux vertébraux des vertèbres adjacentes au disque à remplacer, et un cousin interposé entre les plateaux. Le coussin comporte un corps compressible et une enceinte renfermant un liquide. Cette prothèse a pour inconvénient que le corps compressible est susceptible de s'user, modifiant ainsi à terme le comportement mécanique de la prothèse. De plus, son frottement contre les plateaux provoque l'érosion de particules pouvant migrer dans le corps du patient.

Un but de l'invention est de fournir une prothèse dont le comportement mécanique soit identique sur une longue période de temps, et réduisant les risques de libération de particules dans le corps du patient.

En vue de la réalisation de ce but, on prévoit selon l'invention une prothèse de disque intervertébral comprenant un coussin compressible comportant un corps réalisé dans un matériau, et un liquide apte à venir en contact avec le corps, dans laquelle le liquide et le matériau sont tels que le liquide ne mouille pas le corps.

Sachant que le liquide ne mouille pas le corps, la mise en contact forcée du liquide avec le corps requiert une certaine énergie. Cette énergie est restituée en intégralité lorsqu'on permet au liquide de se séparer à nouveau du corps. On produit ainsi une sorte d'effet ressort. Cet effet est d'autant plus important que la surface de contact du liquide avec le corps est grande. La prothèse peut donc encaisser des efforts très importants, en se déformant en compression ou en flexion, puis produire la déformation inverse en restituant l'énergie emmagasinée. Une telle prothèse peut stocker dans un petit volume une quantité d'énergie considérable. En outre, le mouvement du liquide au contact du corps ne génère aucune usure : la prothèse est donc susceptible de présenter le même comportement mécanique sur une période de temps très longue. De plus, l'absence de frottement entre des pièces solides fait pratiquement disparaître tout risque de libération de particules dans le corps.

L'effet ressort sera très élevé en présence d'un corps poreux même de volume très réduit.

Si l'on prévoit que le corps présente des cavités communiquant entre elles suivant des réseaux très longs, le chemin à parcourir par le liquide lors de l'entrée et de la sortie est particulièrement élevé. Il s'ensuit que le liquide dissipe une partie de l'énergie par sa seule circulation dans le corps. Dès lors, l'ensemble peut constituer un amortisseur partiel. La combinaison de l'effet ressort avec l'effet amortisseur entraîne alors que pour la prothèse, la courbe représentant l'effort en compression en fonction de la variation de longueur de la prothèse suivant son axe principal a une forme en hystérésis, tout comme un disque intervertébral naturel sain. On approche donc de près le comportement mécanique d'un véritable disque.

Avantageusement, la prothèse comporte deux plateaux s'étendant de part et d'autre du coussin, le corps étant fixé à au plus un des plateaux.

D'autres caractéristiques et avantages de l'invention apparaîtront encore dans la description suivante d'un mode préféré de réalisation donné à titre d'exemple non limitatif. Aux dessins annexés :
- la figure 1 est une vue en coupe axiale d'une prothèse selon le mode préféré de réalisation de l'invention ;
- la figure 2 est la courbe montrant la variation de la force de compression appliquée à la prothèse en fonction de l'évolution de sa hauteur suivant son axe ; et
- la figure 3 est une vue en perspective de la prothèse de la figure 1.

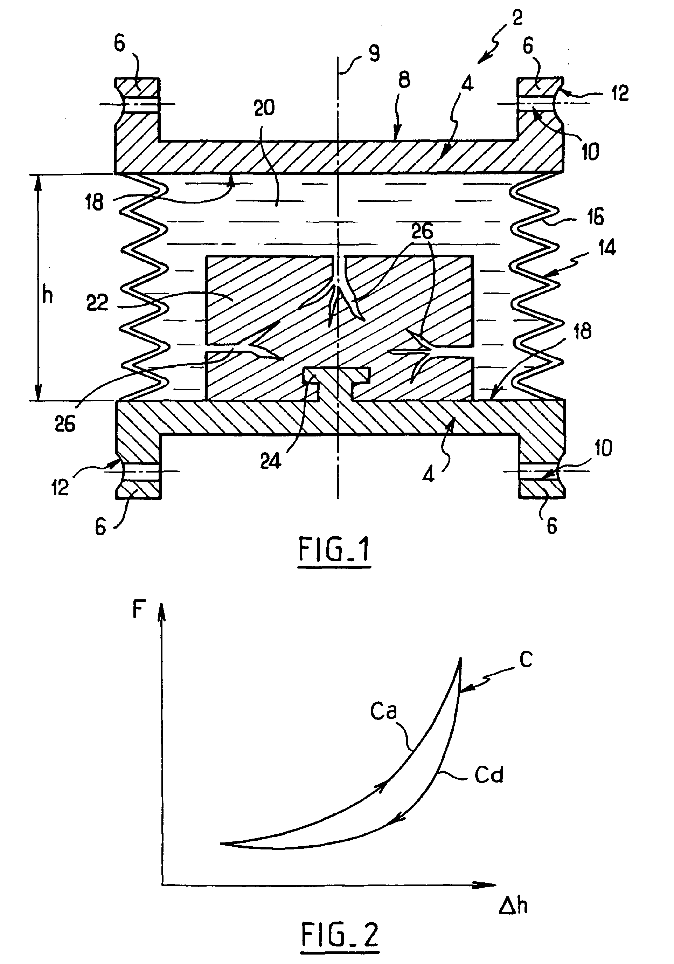

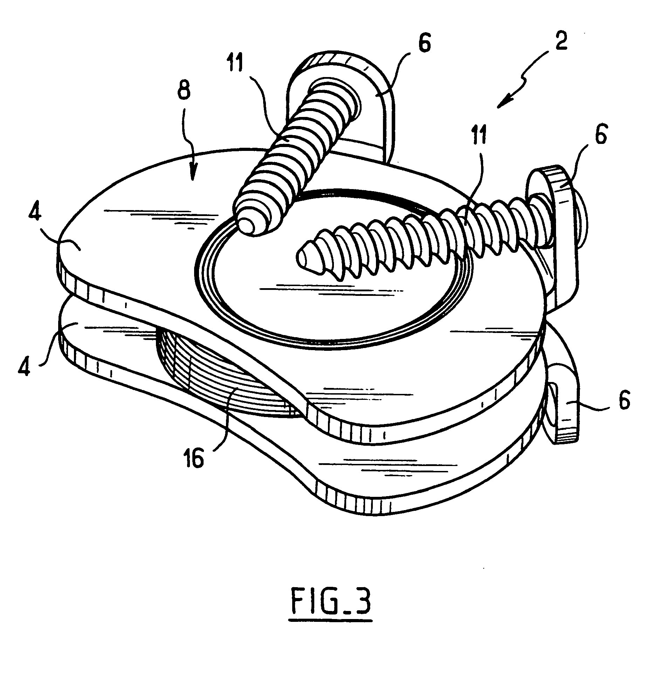

En référence aux figures 1 et 3, la prothèse 2 comprend deux plateaux 4 ayant en plan une forme générale circulaire ou bien de préférence une forme en haricot à hile postérieur. Les deux plateaux sont plats et s'étendent en vis-à-vis parallèlement l'un à l'autre en étant centrés sur un axe principal 9 de la prothèse perpendiculaire aux plateaux.

Chaque plateau 4 comporte deux pattes 6 s'étendant en saillie d'une face externe 8 du plateau 4 perpendiculairement au plan du plateau. Chaque patte 6 présente un orifice 10 la traversant de part en part en direction du centre du plateau et, sur une face de la patte 6 opposée au plateau 4, une empreinte 12 de forme sphérique. Les orifices 10 permettent la réception d'une vis à os 11 ayant une tête dont une face inférieure a une forme sphérique mâle coopérant avec l'empreinte femelle 12 de la patte 6 pour permettre une libre orientation de la vis par rapport à la patte associée. Sur la figure 1, les deux pattes 6 de chaque plateau ont été ramenées dans le même plan de coupe, ce qui n'est pas le cas en réalité, comme le montre la figure 3.

Pour réaliser un ancrage à court terme de la prothèse de disque 2 dans la colonne, on pourra ancrer les vis 11 dans le spondyle des vertèbres adjacentes au disque à remplacer.

Toutefois, on pourra prévoir un ancrage dit à long terme où, en outre, les surfaces 8 des plateaux 4 en contact avec les vertèbres adjacentes sont recouvertes d'hydroxyapatite, ou de toute autre substance connue en soi pouvant stimuler la croissance osseuse. Avant recouvrement, lesdites surfaces 8 pourront être traitées pour obtenir un état de surface plus ou moins poreux, présentant des points d'ancrage pour le tissu osseux, pour assurer une meilleure interface avec ledit tissu osseux.

La prothèse présente un coussin 14 interposé entre les plateaux. Ce coussin comporte un soufflet 16. Il a une forme symétrique de révolution autour de l'axe 9. Sa paroi présente de profil des ondulations permettant de faire varier la longueur du soufflet 16 suivant la direction axiale 9, sans que varie sensiblement la superficie de sa section transversalement à l'axe 9. En l'espèce, ce soufflet, de même que les plateaux 4, est réalisé en titane ou alliage de titane, de sorte qu'il présente une certaine rigidité axiale et forme un ressort de compression. Il peut également être déformé suivant une direction perpendiculaire à l'axe 9 ou subir une torsion autour de l'axe 9 ou d'un axe quelconque perpendiculaire à celui-ci.

Le soufflet 16 présente à ses deux extrémités axiales des bords collés à des bords respectifs des plateaux 4 s'étendant, par exemple mais de façon non illustrée, en saillie d'une face interne 18 des plateaux. Le collage est réalisé de façon étanche de sorte que le soufflet 16 définit avec les deux plateaux 4 une enceinte étanche à volume variable.

Le soufflet 16 présente, par exemple et de façon non illustrée, dix convolutions, soit huit crêtes externes en plus des deux crêtes de fixation aux plateaux 4. Il a ici un diamètre externe d'environ 30 mm et un diamètre interne d'environ 17 mm. Sa hauteur, lorsque la prothèse est hors charge, vaut 10 mm. La paroi du soufflet peut être réalisée au moyen d'une, deux ou trois feuilles chacune de 0,1 mm d'épaisseur et dont la somme des épaisseurs forme l'épaisseur de la paroi. Le soufflet a ici en propre une raideur d'environ 1,6 N/mm.

L'enceinte délimitée par le soufflet 16 et les deux plateaux 4 est remplie d'un liquide 20. Le coussin 14 comporte un corps 22 qui est ici ancré à la face interne plane 18 de l'un des deux plateaux, par exemple le plateau inférieur 4 sur la figure 1. A cette fin, un relief 24 venu du plateau s'étend dans le corps. La forme du corps peut par exemple être cylindrique d'axe 9. Le corps sera par exemple suffisamment étroit perpendiculairement à l'axe 9 pour ne jamais venir en contact avec le soufflet lors de déformations de la prothèse. De même, un espace libre suffisant sera ménagé entre l'extrémité libre, ici supérieure, du corps 22 et le plateau opposé pour qu'ils ne viennent jamais en contact mutuel. Le corps 22 est environné par le liquide apte à venir en contact avec lui.

Le liquide 20 et le matériau du corps 22 seront de préférence biocompatibles. Ils sont choisis de sorte que le liquide soit non mouillant à l'égard du corps, c'est-à-dire qu'en l'absence de sollicitation à cette fin il tende à demeurer hors de contact du corps. Le liquide sera par exemple de l'eau et le matériau du corps un matériau biocompatible tel que du titane ou un alliage de titane. Ce matériau sera de préférence poreux pour offrir une grande surface de contact avec le liquide. De plus, on fera en sorte que les cavités du corps constituant ses pores communiquent entre elles suivant des réseaux assez longs afin de fournir un effet amortisseur en plus de l'effet ressort. Sur la figure 1, les pores ou cavités 26 du corps 22 ont été illustrés de façon schématique pour plus de clarté. Il va de soi que ces pores sont invisibles à l'échelle de la figure 1 et infiniment plus nombreux que ceux illustrés. Ainsi, la hauteur h étant mesurée de l'un à l'autre des plateaux au niveau de l'axe 9, la courbe illustrant sur la figure 2 la variation de la compression F de la prothèse suivant son axe 9 en fonction de sa variation, négative, de hauteur Δh, aura une forme en hystérésis. Ainsi, la courbe Ca indiquant l'évolution de la force F lorsque celle-ci augmente est tout entière au-dessus de la courbe Cd illustrant la décroissance de la force F. Cette forme est due à la combinaison de l'effet ressort et de l'effet amortisseur.

On pourra prévoir une poche de gaz à l'intérieur de l'enceinte de liquide.

La prothèse selon l'invention est particulièrement adaptée à la zone lombaire du rachis.

Bien entendu, on pourra apporter à l'invention de nombreuses modifications sans sortir du cadre de celle-ci. Le corps 22 pourra être sans ancrage à l'égard des plateaux. Il sera ainsi libre de se déplacer spontanément à l'égard de chacun d'eux. La prothèse pourra alors être agencée pour qu'en toute position et tout état de sollicitation de la prothèse, le corps 22 soit en contact avec au plus l'un des plateaux.

Le corps 22 pourra avoir une forme en ellipsoïde.

## Revendications

1. Prothèse de disque intervertébral (2) comprenant un coussin compressible (14) comportant un corps (22) réalisé dans un matériau, et un liquide (20), le corps étant environné par le liquide apte à venir en contact avec le corps (22), **caractérisée en ce que** le liquide et le matériau sont tels que le liquide ne mouille pas le corps (22).

2. Prothèse selon la revendication 1, **caractérisée en ce que** le matériau est poreux.

3. Prothèse selon les revendications 1 à 2, **caractérisée en ce que** la prothèse comporte deux plateaux (4) s'étendant de part et d'autre du coussin (14), le corps (22) étant fixé à au plus un des plateaux.

4. Prothèse selon l'une quelconque des revendications 1 à 3, **caractérisée en ce qu'**elle est agencée de sorte qu'une courbe (c) d'une compression (F) de la prothèse suivant un axe principal (9) de la prothèse en fonction d'une variation (Δh) de dimension (h) de la prothèse suivant cet axe a une forme en hystérésis.

5. Prothèse selon l'une quelconque des revendications 1, 2 ou 4, **caractérisée en ce qu'**elle comprend des plateaux et est agencée de sorte que le corps soit en contact avec au plus un des plateaux.

6. Prothèse selon la revendication précédente, **caractérisée en ce que** le corps est libre de se déplacer par rapport à chaque plateau.

## Claims

1. Intervertebral disc prosthesis (2) comprising a compressible cushion (14) having a body (22) made of a material, and a liquid (20) which is able to come into contact with the body (22), **characterized in that** the liquid and the material are such that the liquid does not wet the body (22).

2. Prosthesis according to Claim 1, **characterized in that** the material is porous.

3. Prosthesis according to Claims 1 and 2, **characterized in that** the prosthesis comprises two plates (4) which extend either side of the cushion (14), the body (22) being fixed to at most one of the plates.

4. Prosthesis according to any one of Claims 1 to 3, **characterized in that** it is designed in such a way that a curve (c) of a compression (F) of the prosthesis along a main axis (9) of the prosthesis as a function of a variation (Δh) in the dimension (h) of the prosthesis along this axis has a hysteresis shape.

5. Prosthesis according to any one of Claims 1, 2 or 4, **characterized in that** it comprises plates and is designed in such a way that the body is in contact with at most one of the plates.

6. Prosthesis according to the proceding claim, **characterized in that** the body is free to move relative to each plate.

## Patentansprüche

1. Bandscheibenprothese (2), die ein komprimierbares Kissen (14) aufweist, welches einen Körper (22) aus einem Material und eine Flüssigkeit (20) enthält, die mit dem Körper (22) in Kontakt kommen kann, **dadurch gekennzeichnet, daß** die Flüssigkeit und das Material derart ausgelegt sind, daß die Flüssigkeit den Körper nicht befeuchtet.

2. Prothese nach Anspruch 1, **dadurch gekennzeichnet, daß** das Material porös ist.

3. Prothese nach einem der Ansprüche 1 oder 2, **dadurch gekennzeichnet, daß** die Prothese zwei Platten (4) aufweist, die sich auf beiden Seiten des Kissens (14) erstrecken, wobei der Körper (22) an höchstens einer der Platten befestigt ist.

4. Prothese nach einem der Ansprüche 1 bis 3, **dadurch gekennzeichnet, daß** sie derart ausgelegt ist, daß ein Graph bzw. eine Kurve (c) einer Kompression (F) der Prothese entlang einer Hauptachse (9) der Prothese als Funktion einer Änderung (Δh) der Abmessung (h) der Prothese entlang dieser Achse die Form einer Hysterese aufweist.

5. Prothese nach einem der Ansprüche 1,2 oder 4, **dadurch gekennzeichnet, daß** sie Platten aufweist und derart ausgelegt ist, daß der Körper mit höchstens einer der Platten in Kontakt ist.

6. Prothese nach einem der vorstehenden Ansprüche, **dadurch gekennzeichnet, daß** sich der Körper bezüglich jeder Platte frei bewegen kann.
